(19) Europäisches Patentamt

European Patent Office

Office européen des brevets

(11) **EP 1 167 491 B1**

(12) **FASCICULE DE BREVET EUROPEEN**

(45) Date de publication et mention
de la délivrance du brevet:
**23.08.2006 Bulletin 2006/34**

(51) Int Cl.:
*C10G 45/32* *(2006.01)* *C07C 7/163* *(2006.01)*

(21) Numéro de dépôt: **01401540.8**

(22) Date de dépôt: **14.06.2001**

(54) **Procédé pour l'hydrogénation de coupes contenant des hydrocarbures**

Verfahren zur Hydrierung von Kohlenwasserstoffschnitten

Process for the hydrogenation of hydrocarbon containing fractions

(84) Etats contractants désignés:
**DE ES GB IT NL**

(30) Priorité: **28.06.2000 FR 0008358**

(43) Date de publication de la demande:
**02.01.2002 Bulletin 2002/01**

(73) Titulaire: **Institut Français du Pétrole**
**92852 Rueil-Malmaison Cedex (FR)**

(72) Inventeurs:
• **Boyer, Christophe**
**69390 Charly (FR)**
• **Coupard, Vincent**
**69120 Vaulx En Velin (FR)**
• **Debuisschert, Quentin**
**92500 Rueil-Malmaison (FR)**

(56) Documents cités:
**EP-A- 0 523 482      EP-A- 0 700 984**
**FR-A- 2 450 867      US-A- 4 113 603**
**US-A- 4 469 907      US-A- 4 704 492**
**US-A- 4 960 960**

Remarques:
Le dossier contient des informations techniques présentées postérieurement au dépôt de la demande et ne figurant pas dans le présent fascicule.

## Description

### Objectif de l'invention :

La présente invention propose un procédé permettant d'hydrogéner une coupe contenant des hydrocarbures comprenant des molécules hautement insaturées contenant au moins deux doubles liaisons ou au moins une triple liaison telles que par exemple des molécules hydrocarbonées telles que le 1,3-Butadiène, le 1,2 Butadiène, le Vinyl acétylène et des molécules d'hydrocarbures contenant 4 atomes de carbones ou plus telles que par exemple du 1-Butène, du 2-Butène, du n-Butane, de l'Iso Butane et/ou de l'Isobutène. Ce procédé s'applique plus particulièrement à l'hydrogénation de coupes contenant des hydrocarbures et notamment du butadiène. Il permet de contrôler la réaction et d'obtenir une haute sélectivité en butène dans le produit sans perte en isobutène et en minimisant l'hydrogénation complète des butènes en butane. Quatre schémas de réalisation particulière du procédé de l'invention sont proposés : le premier et le deuxième permettent d'obtenir une très bonne sélectivité en butène avec une conversion du Butadiène plus faible (entre 80 et 100%), le troisième et le quatrième permettent d'atteindre une conversion proche de 100 % du Butadiène.

### État de l'art :

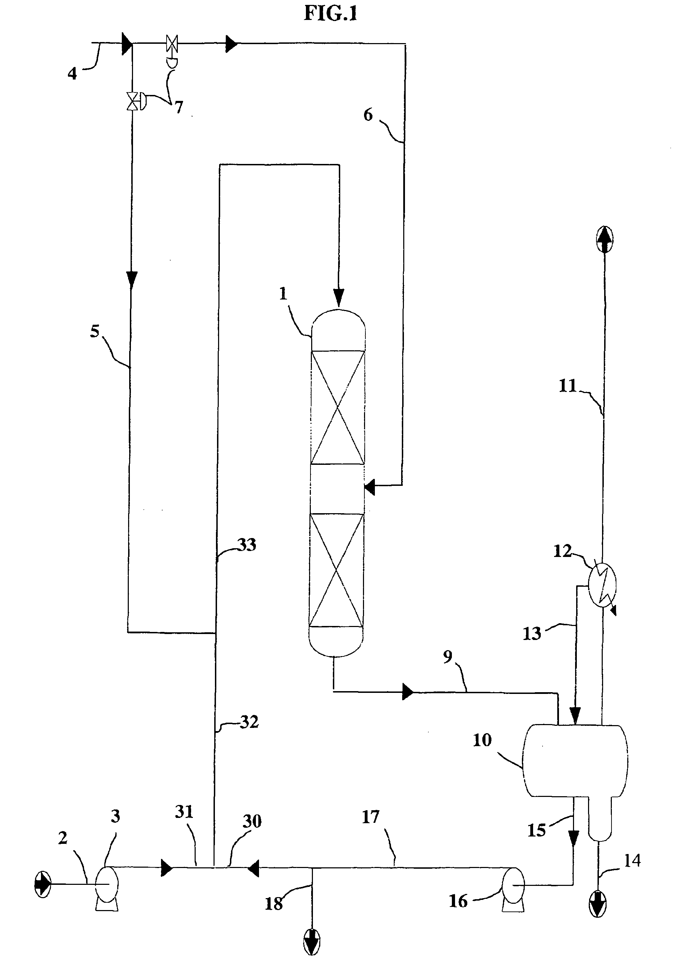

Le brevet WO 93/21137 correspondant au brevet US 5,281,753 propose un procédé d'hydrogénation sélective et d'isomérisation simultanée d'hydrocarbures dans un mélange contenant des molécules insaturées (en particulier des di-oléfines) et ayant 4 atomes de carbone ou plus. L'hydrogénation s'effectue en présence d'hydrogène gazeux à travers un réacteur catalytique à lits fixes étagés. L'hydrogène est introduit dans le réacteur en au moins trois points successifs en augmentant progressivement le ratio molaire d'hydrogène par rapport à la fraction en molécules insaturées. Selon l'enseignement découlant de ce brevet il est proposé d'augmenter ce ratio depuis un ratio sous stoechiométrique au premier point d'injection à un ratio stoechiométrique au deuxième point à enfin un ratio en excès par rapport à la stoechiométrie au dernier point d'injection. Une application particulière décrite dans ce brevet concerne l'hydrogénation du Butadiène au sein d'une coupe d'hydrocarbures contenant en outre du butane et des butènes. Selon l'enseignement de ce brevet les lits catalytiques sont séparés les uns des autres par des lits de matériaux inertes et l'hydrogénation est effectuée telle que par exemple selon le schéma de principe de la figure 1 en courant ascendant de charge et d'hydrogène. Il n'est pas fait mention d'un recyclage d'une partie du produit hydrogéné et de même l'excès d'hydrogène introduit en particulier au niveau du troisième lit n'est pas précisé.

Le brevet US 3,764,633 décrit un procédé d'isomérisation de mono-oléfines contenant une double liaison disposée en extrémité de molécule contenant au moins 4 atomes de carbone et d'hydrogénation sélective simultanée des composés polyinsaturés tels que des dioléfines ou des composés acétyléniques contenus dans la coupe d'hydrocarbures traitée. Le brevet enseigne que le procédé peut mettre en jeux plusieurs lits successifs. Dans ce cas l'injection d'hydrogène est étagée entre les différents lits mais la proportion de l'hydrogène total injecté dans le premier lit est comprise entre 75 et 98 %. L'hydrogène est de préférence introduit avec la charge après vaporisation de ladite charge. La quantité d'hydrogène introduite décroît depuis le premier lit de catalyseur jusqu'au dernier lit de catalyseur. Chaque réacteur contient par exemple deux lits de catalyseurs séparés par une cloison, de l'hydrogène étant introduit avant chacun des lits et dans chacun des lits. L'effluent contenant des hydrocarbures et de l'hydrogène sortant du premier lit n'est pas envoyé directement dans le deuxième lit mais il sort du réacteur et passe dans un échangeur de chaleur avant d'être réintroduit dans le réacteur sous la cloison de séparation des lits au-dessus dudit deuxième lit. Ce brevet n'enseigne pas le recyclage d'une fraction du produit hydrogéné et par ailleurs lorsqu'un réacteur fonctionne, l'autre est en phase de régénération du catalyseur.

Le brevet US 4,260,840 concerne l'hydrogénation sélective du Butadiène d'une coupe C4. Le produit contient au moins 30 % en poids de Butène-1 et du Butadiène à l'état de traces. Cette charge est hydrogénée de façon à minimiser l'isomérisation du butene-1 et à minimiser l'hydrogénation de butènes en butane. Il n'est pas fait mention d'injection étagée d'hydrogène ni de recyclage d'une fraction du produit hydrogéné.

Le brevet US 4,960,960 décrit un procédé d'hydrogénation contenant deux zones qui peuvent comprendre un ou deux lits successifs chacun pouvant être alimenté par la charge gazeuse. Le procédé n'est pas spécifique à une réaction d'hydrogénation particulière et ce brevet n'enseigne pas l'introduction étagée d'hydrogène avant chacun des lits de catalyseur. Enfin selon l'enseignement de ce brevet la phase liquide passe dans un distributeur statique contenu dans le réacteur lui-même, l'hydrogène arrivant par une autre entrée en dessus dudit distributeur statique et il n'est pas fait mention de la possibilité d'employer un mélangeur statique pour améliorer le mélange de la phase gazeuse contenant de l'hydrogène et de la coupe liquide contenant des hydrocarbures.

Le brevet US 4,469,907 ayant la même priorité que la demande de brevet EP 87980 décrit une méthode d'hydrogénation de coupe pétrolière contenant des molécules insaturées à 4 atomes de carbone ou plus en utilisant un réacteur à lit fixe dans lequel l'hydrogène est injecté en 2 ou 3 points successifs. Les auteurs précisent que la quantité d'hydrogène injecté au 2ème ou 3ème point d'injection correspond à une fraction entre 5 et 100 % de la quantité injectée au point précédent. L'excès d'hydrogène par rapport à la stoechiométrie est compris entre 10 et 100 %. Aucune performance en terme de conversion ou sélectivité n'est proposée. Ce brevet n'enseigne pas le recyclage d'une partie du produit hydrogéné issu du premier réacteur.

Le brevet EP 0523 482 décrit un procédé d'hydrogénation sélective du Butadiène en phase liquide seule ou en écoulement ruisselant. Les charges pétrolières traitées contiennent entre 20 et 80 % poids de Butadiène. Le procédé contient deux sections réactionnelles : à la sortie de la première zone le résidu en Butadiène dans le produit est compris entre 0,1 et 20 %poids et en sortie de la deuxième zone il est compris entre 0,005 et 1 % poids. De façon générale le brevet prévoit un rapport 5 entre la teneur en Butadiène à la sortie de la première zone et la teneur à la sortie de la deuxième zone. Le brevet prévoit une sélectivité en butènes supérieure à 96 %. Les conditions opératoires sont précisées : de 40 à 120 °C pour la température, de 5 à 50 bar pour la pression et de 0,1 à 30 h$^{-1}$ pour la vitesse volumétrique horaire. Il n'est pas fait mention d'injection étagée d'hydrogène dans le réacteur. Ce brevet enseigne le recyclage d'une partie du produit hydrogéné et l'utilisation de deux réacteurs séparés en série avec une quantité d'hydrogène fourni à chacune des deux zones de une à deux fois la quantité stoechiométrique nécessaire. Il n'enseigne pas la possibilité de recycler une partie du produit sortant du premier réacteur en tête de celui-ci. La quantité de produit hydrogéné recyclée n'est pas mentionnée.

Le brevet EP 700 984 décrit un procédé d'hydrogénation d'une coupe d'hydrocarbures contenant de 2 à 20 atomes de carbone contenant des hydrocarbures mono insaturés et au moins un hydrocarbure poly insaturé. La charge à traiter est au moins en partie liquide et circule à travers un réacteur contenant au moins un lit fixe et au moins un mélangeur statique situé à l'amont de la section de sortie. Le brevet précise que les lits peuvent être étagés dans un même réacteur avec la possibilité d'injecter de l'hydrogène entre deux étages de lits. Un recyclage partiel du produit peut éventuellement être effectué, mais la quantité de produit recyclée n'est pas précisée. Par ailleurs ce brevet n'enseigne pas l'utilisation de deux réacteurs successifs comprenant chacun deux lits de catalyseurs ni dans ce cas le recyclage d'une partie du produit liquide issu du premier réacteur et obtenu après séparation d'une phase gazeuse et élimination d'une phase aqueuse qu'il contient.

**Présentation de l'invention :**

Dans sa définition la plus large la présente invention concerne un procédé pour l'hydrogénation d'une coupe liquide contenant des hydrocarbures et notamment des molécules insaturées contenant au moins deux doubles liaisons ou au moins une triple liaison dans lequel les molécules insaturées contenant au moins deux doubles liaisons ou au moins une triple liaison sont au moins partiellement hydrogénées en molécules moins insaturées contenant au moins une double liaison, dans au moins un réacteur comprenant au moins deux lits distincts d'au moins un catalyseur d'hydrogénation, et dans lequel une phase gazeuse contenant de l'hydrogène est introduite pour partie en mélange avec ladite coupe avant le premier lit de catalyseur et pour partie avant les lits suivants contenus dans ledit réacteur, et dans lequel dans le premier réacteur l'hydrogénation est effectuée à courant descendant de liquide et de gaz et dans lequel le mélange gaz/liquide issu dudit premier réacteur est envoyé dans une section de séparation à partir de laquelle on récupère une phase gazeuse, une phase aqueuse que l'on élimine et une phase liquide contenant le produit hydrogéné issu dudit premier réacteur qui est au moins en partie renvoyé en mélange avec ladite coupe à l'entrée dudit premier réacteur avant le premier lit de catalyseur contenu dans ledit premier réacteur, le taux de recyclage, dans le premier réacteur, égal au rapport du débit volumique de recycle sur le débit volumique de charge entrant dans le lit réacteur est compris entre 10:1 et 30:1.

Le procédé de l'invention s'applique en particulier à l'hydrogénation d'une coupe contenant des hydrocarbures qui est une coupe contenant essentiellement des hydrocarbures et qui contient du butadiène. Au sens de la présente description le terme essentiellement signifie que la coupe contient au moins 80 %, souvent au moins 95 % et le plus souvent au moins 98 % en masse d'hydrocarbures par rapport à la masse totale de la coupe.

L'invention s'applique particulièrement bien à l'hydrogénation sélective d'une coupe pétrolière riche en butadiène, c'est à dire que la charge d'hydrocarbures à traiter contient en général entre 20 et 95% en masse de Butadiène, souvent de 25 à 75 % en masse de butadiène et préférentiellement entre 30 et 50 % en masse de Butadiène. Cette hydrogénation peut s'effectuer en écoulement gaz-liquide en régime noyé avec un taux de vaporisation volumique compris entre 5 et 30 % et de préférence entre 5 et 20 % ou en écoulement liquide seul, avec un taux de vaporisation compris entre 0 et 5 %. On choisit préférentiellement d'effectuer la réaction en phase liquide seule. Le procédé de la présente invention est habituellement mis en oeuvre dans au moins un réacteur à lit fixe, de préférence de 1 à 2 réacteurs. Chaque réacteur contenant une superposition d'au moins deux lits, de préférence de 2 à 4 lits et de manière plus préférée de 2 lits qui sont alimentés indépendamment en charge gazeuse. Le taux de recyclage de la phase liquide contenant le produit hydrogéné issu du premier réacteur dans ledit premier réacteur, égal au rapport du débit volumique de recycle sur le débit volumique de charge entrant dans ledit réacteur est le plus souvent compris entre 15 : 1 et 25 : 1.

Le premier réacteur contient deux lits successifs de catalyseur et la répartition du débit de phase gazeuse contenant de l'hydrogène injectée en mélange avec la coupe liquide en tête dudit premier réacteur avant le premier lit de catalyseur est de 50 à 70 % en mole par rapport à la somme du débit molaire total de phase gazeuse injectée dans le réacteur. De manière plus précise le premier réacteur contient deux lits de catalyseur superposés et la répartition du débit de charge gazeuse injecté dans le premier réacteur entre la ligne d'entrée et la ligne d'injection secondaire est comprise pour ce qui concerne la ligne d'entrée entre environ 50 et 70 % en mole par rapport à la somme du débit molaire injecté dans le réacteur, et pour ce qui concerne la ligne d'injection secondaire avant le deuxième lit de catalyseur entre 30 et 50 % en mole par rapport à la somme du débit molaire injecté dans le réacteur. Au niveau de l'injection de la charge gazeuse en tête du réacteur la proportion molaire en hydrogène est en dessous de la stoechiométrie par rapport à tous les composés C4 polyinsaturés comprenant au moins une triple liaison et/ou au moins 2 doubles liaisons, cette proportion est comprise entre 0,5 et 1 mole d'hydrogène par mole de composé C4 polyinsaturé à traiter. Après l'injection secondaire, la proportion molaire d'hydrogène est supérieure à la stoechiométrie par rapport à tous les composés C4 polyinsaturés comprenant au moins une triple liaison et/ou au moins 2 double liaisons, cet excès par rapport à la stoechiométrie est de préférence compris entre 1 et 30 % et de manière plus préférée entre 1 et 20 %. Selon une réalisation particulière de l'invention la charge liquide et la phase gazeuse introduites en tête du premier réacteur traversent avant ladite introduction dans ledit réacteur un mélangeur statique.

Selon une forme de réalisation particulière la phase gazeuse issue de la section de séparation est envoyée dans une section de refroidissement à partir de laquelle on récupère une phase liquide que l'on recycle dans la section de séparation

La pression en entrée de réacteur est comprise en général entre 1 et 3 MPa, et le plus souvent entre 2 et 2,5 MPa. La température en entrée du réacteur est en général comprise entre 30 et 60 °C et de préférence entre 35 et 45 °C. La vitesse volumétrique horaire, calculée par rapport au débit de charge en entrée est comprise en général entre 3 et 10 h$^{-1}$ (exprimé en m$^3$ à 15 °C de charge liquide à traiter par m$^3$ de catalyseur et par heure) et de façon préférentielle, entre 3,5 et 7,5 h$^{-1}$

Les catalyseurs d'hydrogénation utilisés sont identiques ou différents dans chaque lit catalytique de chacun des réacteurs et comprennent de préférence sur un support minéral de préférence au moins un métal noble du groupe VIII, de manière plus préférée du palladium sur alumine.

Le catalyseur utilisé dans le premier réacteur contient de manière très préférée 0,2 % en poids de palladium associé le plus souvent à un ou plusieurs dopants qui peuvent être par exemple choisis dans le groupe formé par : l'or, l'argent et le cuivre en une quantité généralement de 1 à 10000 ppm en poids, souvent de 1 à 5000 ppm en poids et le plus souvent d'environ de 1 à 1000 ppm en poids de préférence sur un support d'alumine à haute pureté. Ce premier réacteur contient de manière préférée deux lits de catalyseur identique (même métal noble, même quantité de métal noble et même support). Lorsque l'on utilise deux réacteurs en série le catalyseur utilisé dans le deuxième réacteur contient habituellement une quantité de métal noble plus importante que celle du catalyseur contenu dans les lits catalytiques contenus dans le premier réacteur. Par exemple ce catalyseur contient 0,3 % en poids de palladium de préférence sur un support d'alumine à haute pureté. La charge gazeuse est souvent composée d'un mélange d'hydrogène et d'au moins un deuxième gaz inerte pour la réaction ; ce deuxième gaz peut être par exemple choisi dans le groupe formé par le méthane, le propane, le butane, l'azote, l'argon, le monoxyde de carbone et le dioxyde de carbone. Ce deuxième gaz est généralement majoritairement du méthane ou du propane. La proportion molaire d'hydrogène dans la charge gazeuse est comprise en général entre 60 % et 99,99 % et le plus souvent entre 80 % et 99,99 %, le complément à 100 % étant l'un des gaz inertes cités ci-devant.

Les performances de ce procédé intégrant un seul réacteur permettent d'obtenir une teneur en Butadiène à la sortie du réacteur (1), habituellement comprise entre 0,1 et 5 % en poids. La sélectivité η en butènes dans la réaction de l'hydrogène sur le Butadiène (définie par l'équation (1) ci-après) est en général comprise entre 90 et 100 % et le plus souvent entre 95 et 100 %. La conversion en Butadiène entre l'entrée du réacteur et sa sortie est en général comprise entre 70 et 100 %.

$$\eta = \frac{\sum Butènes_{sortie} - \sum Butènes_{entrée}}{Butadiène_{entrée} - Butadiène_{sortie}} \qquad (1)$$

Le procédé de la présente invention peut intégrer un deuxième réacteur en série du premier réacteur dont les caractéristiques sont décrites au paragraphe précédent. Dans le cas où ce deuxième réacteur contient deux lits successifs (superposés) le débit gazeux injecté en amont du premier lit est en général compris entre 50 et 70 % du débit molaire total injecté dans ledit deuxième réacteur et le débit gazeux injecté en amont du deuxième lit dudit deuxième réacteur est en général compris entre 30 et 50 % du débit molaire total injecté dans ledit deuxième réacteur et le plus souvent cette répartition est de 50 % et 50 % pour les deux points d'injection. Au niveau de l'injection de la charge gazeuse en entrée du deuxième réacteur (en amont du premier lit de catalyseur contenu dans ledit deuxième réacteur) la proportion molaire en hydrogène est de préférence en dessous ou égale à la stoechiométrie par rapport à tous les composés C4 polyinsaturés comprenant au moins une triple liaison et/ou au moins 2 double liaisons : cette proportion est comprise entre 0,5 et 1 mole d'hydrogène par mole de composé C4 polyinsaturé à traiter. Au niveau de l'injection secondaire, en amont du deuxième lit, la proportion molaire d'hydrogène est de préférence supérieure à la stoechiométrie par rapport à tous les composés C4 polyinsaturés comprenant au moins une triple liaison et/ou au moins 2 doubles liaisons : cet excès est de préférence compris entre 10 et 200 % et de manière plus préférée compris entre 30 et 150 %. La température en entrée du réacteur est en général comprise entre 30 et 60 °C et de préférence comprise entre 35 et 45°C. La vitesse volumétrique horaire, calculée par rapport au débit de charge en entrée est en général comprise entre 3 et 10 $h^{-1}$ et de façon préférentielle, entre 4,5 et 7,5 $h^{-1}$. Les performances de ce procédé, comprenant deux réacteurs en série, permettent d'obtenir à la sortie du premier réacteur une teneur en Butadiène généralement comprise entre 0,1 et 5 % en poids et le produit final issu du deuxième réacteur contient généralement une teneur en Butadiène comprise entre 10 ppm et 1 % en poids. La sélectivité en butènes η dans la réaction de l'hydrogène sur le Butadiène est généralement comprise entre 90 et 100 % à la sortie du premier réacteur et entre 95 et 100 % à la sortie du deuxième réacteur. La conversion du Butadiène est généralement comprise entre 95 et 100 % sur l'ensemble du procédé. Le plus souvent lorsque l'hydrcgénation est effectuée dans deux réacteurs successifs le premier travaille à courant descendant de liquide et de gaz alors que le deuxième travaille à courant ascendant de liquide et de gaz et chacun desdits réacteurs contient :

- au moins deux lits successifs de catalyseur,
- une introduction avant chacun des lits de catalyseur d'une phase gazeuse contenant de l'hydrogène correspondant à une partie de la phase gazeuse totale introduite dans lesdits réacteurs,
- une introduction d'une phase liquide correspondant à une partie du produit hydrogéné issue dudit premier réacteur.

Les figures annexées proposent des schémas non limitatifs pour la mise en oeuvre du procédé de l'invention.

**Description de la figure n°1 :**

Le schéma réactionnel comprend un réacteur à lit fixe (1). Ce réacteur (1) est un réacteur à lit fixe à deux lits superposés qui fonctionne en écoulement descendant co-courant. La charge liquide (2) est injectée au travers d'une pompe (3) en tête du réacteur par les lignes (31), (32) et (33). La charge gazeuse (4) est injectée à deux niveaux en amont du réacteur (1) : au niveau de la ligne d'injection de la charge liquide par les lignes (5) et (33) et au niveau intermédiaire du réacteur, entre les deux lits successifs par la ligne (6). La répartition des débits de l'injection gazeuse entre la ligne (5) et la ligne (6) est contrôlée à l'aide de deux vannes de régulation (7). Ce mélange peut éventuellement traverser un échangeur de chaleur soit au moment du démarrage soit tout au long de l'opération de l'unité (échangeur monté en by-pass par rapport à la conduite d'entrée principale non représenté sur la figure 1). En sortie du réacteur (1), le mélange gaz/liquide issu du réacteur est injecté au travers de la ligne (9) dans un séparateur haute pression (10). Les effluents gazeux (11) du séparateur (10) traversent alors un condenseur (12) pour récupérer en phase liquide (après condensation) les composés de la coupe C4 contenu dans la phase gazeuse. Cette phase condensée regagne alors le séparateur par la ligne (13). En partie basse du séparateur la fraction eau est évacuée en (14). En partie basse intermédiaire, l'effluent liquide (15) est renvoyé à travers une pompe de recycle (16) vers l'entrée du réacteur (1) par les lignes (17), (30), (32) et (33) Une fraction de ce débit de recycle est dérivé par la ligne (18) pour constituer le produit de la réaction.

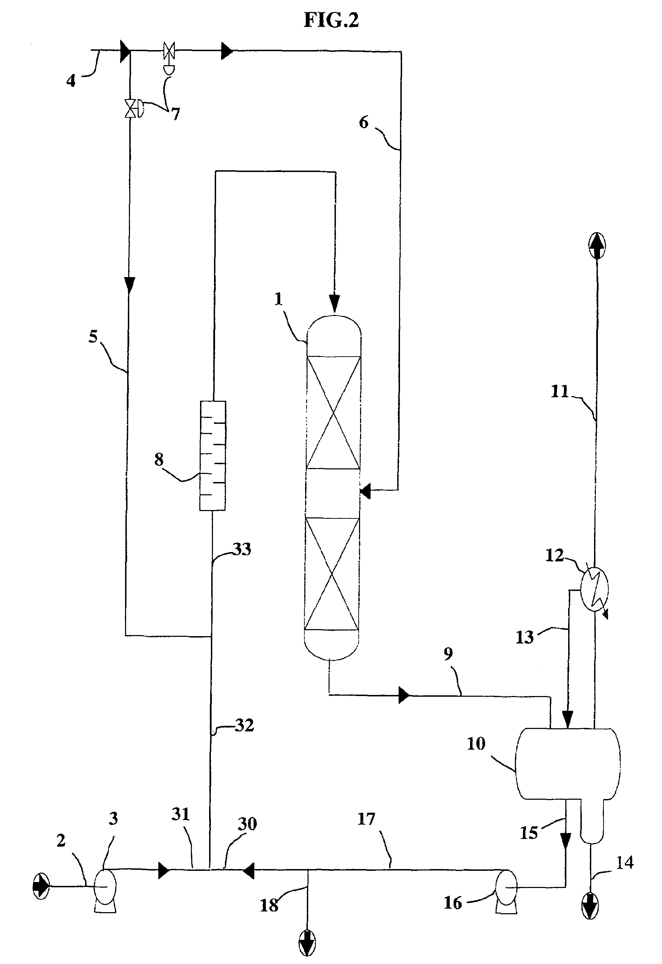

**Description de la figure n°2 :**

Le schéma réactionnel est identique à celui représenté sur la figure 1 à l'exception du fait qu'en amont de la tête du réacteur le mélange gaz/liquide (ligne (33)) traverse un mélangeur statique (8) du même type que ceux décrits dans le brevet EP 700 984.

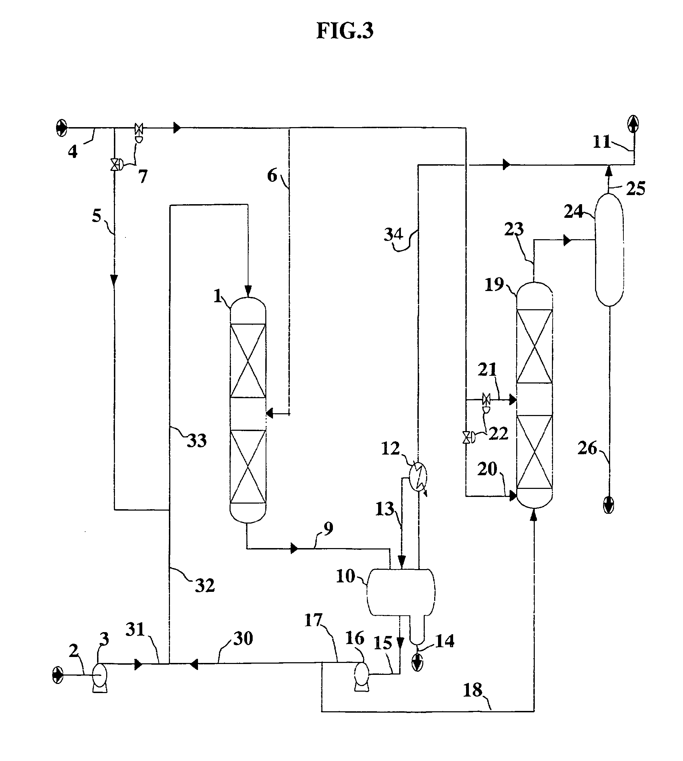

**Description de la figure n°3 :**

Ce schéma comprend un premier réacteur à lit fixe (1) qui contient deux lits superposés et qui fonctionne en écoulement descendant co-courant (gaz-liquide). Le deuxième réacteur (19) est un réacteur à lit fixe à deux lits superposés fonctionnant en écoulement ascendant. La charge liquide (2) est injectée au travers d'une pompe (3) en tête du réacteur (1) par les lignes (31), (32) et (33). La charge gazeuse (4) est injectée à deux niveaux en amont du réacteur (1) : au niveau de la ligne d'injection de la charge liquide par les lignes (5) et (33) et au niveau intermédiaire du réacteur, entre les deux lits successifs par la ligne (6). La répartition des débits de l'injection gazeuse entre la ligne (5) et la ligne (6) est contrôlée à l'aide de deux vannes de régulation (7). Ce mélange peut traverser un échangeur de chaleur soit au moment du démarrage soit tout au long de l'opération de l'unité (échangeur monté en by-pass par rapport à la conduite d'entrée principale non représenté sur la figure 3). En sortie du réacteur (1), le mélange gaz/liquide issu du réacteur est injecté au travers de la ligne (9) dans un séparateur haute pression (10). Les effluents gazeux (11) du séparateur (10) traversent alors un condenseur (12) pour récupérer en phase liquide (après condensation) les composés de la coupe C4 contenu dans la phase gazeuse. Cette phase condensée regagne alors le séparateur par la ligne (13). En partie basse du séparateur la fraction eau est évacuée en (14). En partie basse intermédiaire, l'effluent liquide (15) est renvoyée à travers une pompe de recycle (16) vers l'entrée du réacteur (1) par les lignes (17), (30), (32) et (33). Une fraction de ce débit de recycle est dérivé sur la ligne (18) pour alimenter le réacteur (19).

Dans le réacteur (19) la charge liquide est injectée en partie basse du réacteur et la charge gazeuse, de même composition que celle injectée dans le réacteur (1), est injectée à deux niveaux : en amont du premier lit par les lignes (34) et (20) et en amont du deuxième lit par la ligne (34) et (21). La répartition du débit gazeux injecté en (20) et en (21) est contrôlée par deux vannes de régulation (22). En sortie du réacteur (19) l'effluent est récupéré par la ligne (23) et travers un organe (24) destiné à séparer la fraction la plus légère des produits sortant dudit organe par la ligne (25), (coupe C1 à C3 et hydrogène) qui rejoint dans la ligne (11) les effluents gazeux issus du ballon séparateur (10). La phase liquide constitue le produit de la réaction et est récupérée par la ligne (26).

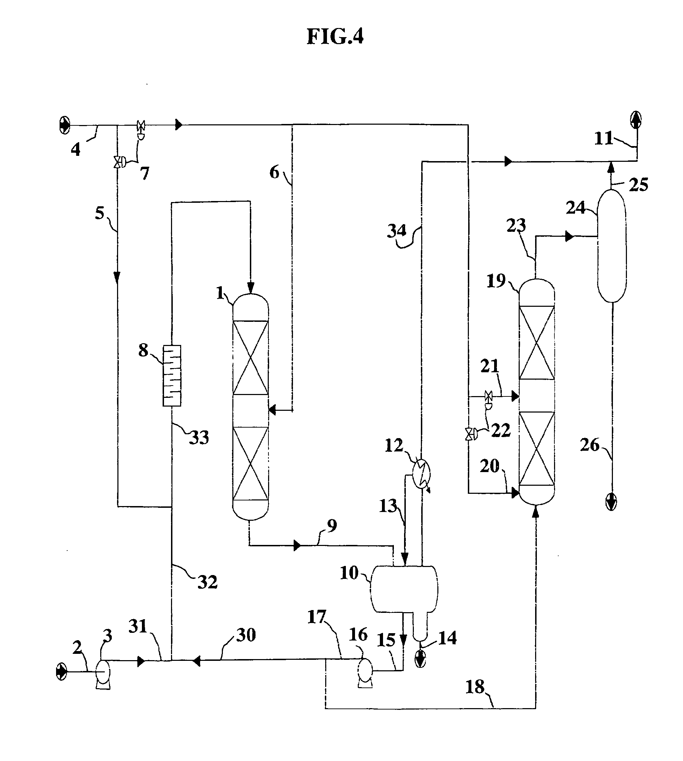

**Description de la figure n°4 :**

Le schéma réactionnel est identique à celui représenté sur la figure 3 à l'exception du fait qu'en amont de la tête du réacteur le mélange gaz/liquide (lignes (33)) traverse un mélangeur statique (8) du même type que ceux décrits dans le brevet EP 700 984.

**Exemple n°1 :**

Hydrogénation partielle d'une coupe pétrolière C4 riche en Butadiène. Les compositions de la charge à traiter, de la phase liquide en entrée du réacteur et du produit sont données dans le tableau 1. Le catalyseur contient 0,2 % poids de Palladium sur un support d'alumine de haute pureté pour les deux lits du réacteur, la surface spécifique du catalyseur est de 70 $m^2$/g. Le schéma réactionnel correspond au schéma de la figure n° 2. La pression en entrée du réacteur est de 1,3 MPa, la température de 43 °C. Le rapport du débit massique de recycle sur le débit massique de la charge à traiter est de 17. La vitesse volumique horaire correspondant au rapport du débit volumique de charge à 15°C sur le volume de catalyseur, est de 4 $h^{-1}$ ($m^3$/$m^3$ catalyseur/h.). La répartition de la charge gazeuse entre l'entrée et le milieu du réacteur est de 50 % pour l'entrée et 50 % pour le niveau intermédiaire. La proportion molaire en hydrogène dans la charge gazeuse est de 99,8 %. La sélectivité η en butènes par rapport au butadiène est de 87,8 %. Le taux de butadiène en sortie est de 0,29 % poids. La conversion en Butadiène au niveau du réacteur est de 90,2 %.

**Tableau 1: Composition de la charge, de l'écoulement en entrée réacteur et du produit dans l'exemple n°1**

| | Charge | Entrée réacteur | Produit |
|---|---|---|---|
| **Composants** | % poids | % poids | % poids |
| **1-3 Butadiène** | 45,20 | 2,96 | 0,29 |
| **1-2 Butadiène** | <0.1 | <0,1 | <0,1 |
| **Vinyl acétylène** | 1,64 | 0,10 | <0,1 |
| **Ethyl acétylène** | <0,1 | <0,1 | <0,1 |
| **Isobutène** | 20,79 | 21,10 | 21,12 |
| **1-Butène** | 13,59 | 16,92 | 17,13 |
| **Cis-2-Butène** | 3,99 | 14,63 | 15,30 |
| **Trans-2-butène** | 6,14 | 33,07 | 34,77 |
| **Isobutane** | 4,51 | 4,74 | 4,75 |
| **N-butane** | 4,13 | 6,48 | 6,63 |

**Exemple n°2 :**

Hydrogénation d'une coupe pétrolière riche en Butadiène. Les compositions de la charge, de l'écoulement d'entrée et du produit du réacteur (1) sont données dans le tableau 2. Le schéma réactionnel correspond au schéma de la figure n° 4. Le catalyseur contient 0,2 % poids de Palladium sur un support d'alumine de haute pureté pour les deux lits du premier réacteur et 0,3 % poids de Palladium sur un support d'alumine de haute pureté pour les deux lits du deuxième réacteur. Les surfaces spécifiques des catalyseurs sont de 70 m$^2$/g. A l'entrée du réacteur (1), la pression à est de 2,41 MPa et la température de 40 °C. Le rapport du débit massique de recycle sur le débit massique de la charge à traiter est de 22. La vitesse volumique horaire correspondant au rapport du débit volumique de charge à 15°C sur le volume de catalyseur, est de 3,8 h$^{-1}$ (m$^3$/m$^3$ catalyseur/h.) La répartition en charge gazeuse entre l'entrée et le milieu du lit est de 55 % en entrée et de 45 % en milieu de réacteur. La concentration molaire en hydrogène dans la charge gazeuse est de 93 % le reste étant du méthane. La sélectivité η en butènes par rapport au butadiène transformé pour le réacteur (1) est de 98,4 % et le pourcentage de butadiène en sortie du réacteur (1) est de 1,3 % poids.

A l'entrée du réacteur (19), la pression est de 2,6 MPa et la température est de 35 °C. La composition de l'écoulement liquide en entrée et du produit du réacteur(19) sont présentées dans le tableau 3. La répartition en charge gazeuse entre l'entrée et le milieu du réacteur est de 60 % en entrée et 40 % en milieu de réacteur. La vitesse volumique horaire correspondant au rapport du débit volumique de charge à 15°C sur le volume de catalyseur, est de 6 h$^{-1}$. Le gaz de rejet contient 42% en poids de méthane et 51 % en poids de butènes. La sélectivité en butènes par rapport au Butadiène total transformé dans les deux réacteurs est de 97 %. La teneur en Butadiène en sortie du réacteur (19) est inférieur à 100 ppm. La conversion du Butadiène sur l'ensemble du schéma est de 100 %.

**Tableau 2 : Composition de la charge, de l'écoulement en entrée et du produit du réacteur (1) de l'exemple n°2**

| | Charge | Entrée réacteur 1 | Produit |
|---|---|---|---|
| Constituant | % poids | % poids | % poids |
| 1-3 Butadiène | 48,53 | 3,07 | 0,98 |
| 1-2 Butadiène | 0,16 | 0,02 | 0,01 |
| Vinyl acétylène | 0,61 | 0,03 | <0,01 |
| Ethyl acétylène | 0,15 | 0,01 | <0,01 |
| Isobutène | 24,54 | 24,93 | 24,95 |
| 1-Butène | 12,91 | 39,41 | 40,63 |
| Cis-2-Butène | 3,95 | 7,45 | 7,61 |
| Trans-2-butène | 5,15 | 20,39 | 21,09 |
| Isobutane | 0,61 | 0,65 | 0,65 |
| N-butane | 3,39 | 4,06 | 4,09 |

**Tableau 3 : Composition de l'écoulement en entrée et du produit du réacteur (19) de l'exemple n°2**

| | Entrée réacteur 2 | Produit |
|---|---|---|
| Constituant | % poids | % poids |
| 1-3 Butadiène | 0,98 | <0,01 |
| 1-2 Butadiène | 0,01 | <0,01 |
| Vinyl acétylène | <0,01 | <0,01 |
| Ethyl acétylène | <0,01 | <0,01 |
| Isobutène | 24,95 | 24,80 |
| 1-Butène | 40,63 | 35,94 |
| Cis-2-Butène | 7,61 | 9,35 |
| Trans-2-butène | 21,09 | 23,66 |
| Isobutane | 0,65 | 0,66 |
| N-butane | 4,09 | 5,60 |

**Revendications**

1. Procédé pour l'hydrogénation d'une coupe liquide contenant des hydrocarbures et notamment des molécules insaturées contenant au moins deux doubles liaisons ou au moins une triple liaison **caractérisé en ce que** les molécules insaturées contenant au moins deux doubles liaisons ou au moins une triple liaison sont au moins partiellement hydrogénées en molécules moins insaturées contenant au moins une double liaison, dans au moins un réacteur comprenant au moins deux lits distincts d'au moins un catalyseur d'hydrogénation, **en ce qu'**une phase gazeuse contenant de l'hydrogène est introduite pour partie en mélange avec ladite coupe avant le premier lit de catalyseur et pour partie avant les lits suivants contenus dans ledit réacteur, **en ce que** dans le premier réacteur l'hydrogénation est effectuée à courant descendant de liquide et de gaz et **en ce que** le mélange gaz/liquide issu dudit premier réacteur est envoyé dans une section de séparation à partir de laquelle on récupère une phase gazeuse, une phase aqueuse que l'on élimine, et une phase liquide contenant le produit hydrogéné issu dudit premier réacteur qui est au moins en partie renvoyé en mélange avec ladite coupe à l'entrée dudit premier réacteur avant le premier lit de catalyseur contenu dans ledit premier réacteur, et **en ce que** le taux de recyclage, dans le premier réacteur, égal au rapport du débit volumique de recycle sur le débit volumique de charge entrant dans ledit réacteur est compris entre 10 : 1 et 30 : 1.

2. Procédé selon la revendication 1 dans lequel l'hydrogénation s'effectue en écoulement gaz-liquide en régime noyé avec un taux de vaporisation volumique compris entre 5 et 30 %.

3. Procédé selon la revendication 1 dans lequel l'hydrogénation s'effectue en écoulement liquide seul, avec un taux de vaporisation compris entre 0 et 5 %.

4. Procédé selon l'une des revendications 1 à 3 dans lequel la coupe contenant des hydrocarbures est une coupe contenant essentiellement des hydrocarbures contenant du butadiène.

5. Procédé selon la revendication 4 dans lequel la coupe contient du butadiène en une proportion massique de 20 % à 95 %.

6. Procédé selon l'une des revendications 1 à 5 dans lequel le taux de recyclage, dans le premier réacteur, égal au rapport du débit volumique de recycle sur le débit volumique de charge entrant dans ledit réacteur est compris entre 15 : 1 et 25 : 1.

7. Procédé selon l'une des revendications 1 à 6 dans lequel le premier réacteur contient deux lits successifs de catalyseur et la répartition du débit de phase gazeuse contenant de l'hydrogène injectée en mélange avec la coupe liquide en tête dudit premier réacteur avant le premier lit de catalyseur est de 50 à 70 % en mole par rapport à la somme du débit molaire total de phase gazeuse injectée dans le réacteur.

8. Procédé selon l'une des revendications 1 à 7 dans lequel la charge liquide et la phase gazeuse introduites en tête du premier réacteur traversent avant ladite introduction dans ledit réacteur un mélangeur statique.

9. Procédé selon rune des revendications 1 à 8 dans lequel l'hydrogénation est effectuée dans deux réacteurs successifs le premier travaillant à courant descendant de liquide et de gaz et le deuxième travaillant à courant ascendant de liquide et de gaz contenant :

   - au moins deux lits successifs de catalyseur,
   - une introduction avant chacun des lits de catalyseur d'une phase gazeuse contenant de l'hydrogène correspondant à une partie de la phase gazeuse totale introduite dans lesdits réacteurs,
   - une introduction d'une phase liquide correspondant à une partie du produit hydrogéné issue dudit premier réacteur.

10. Procédé selon la revendication 9 dans lequel le deuxième réacteur contient deux lits successifs de catalyseur et le débit de la phase gazeuse contenant de l'hydrogène injectée avant le premier lit de catalyseur est de 50 à 70 % par rapport au débit molaire total de phase gazeuse injectée dans ledit réacteur.

11. Procédé selon l'une des revendications 1 à 10 dans lequel la phase gazeuse issue de la section de séparation est envoyée dans une section de refroidissement à partir de laquelle on récupère une phase liquide que l'on recycle dans la section de séparation

**12.** Procédé selon l'une des revendications 1 à 11 dans lequel les catalyseurs d'hydrogénation utilisés sont identiques ou différents dans chaque lit catalytique de chacun des réacteurs et comprennent sur un support minéral au moins un métal noble du groupe VIII, de préférence du palladium sur alumine.

**13.** Procédé selon la revendication 12 dans lequel le premier réacteur comprend deux lits de catalyseur identiques contenant le même métal noble du groupe VIII sur un même support minéral dans une proportion identique.

**14.** Procédé selon la revendication 12 ou 13 dans lequel le catalyseur contient un agent dopant choisi dans le groupe formé par l'or, l'argent et le cuivre en une quantité d'environ 1 à environ 10000 ppm en poids par rapport au support.

**15.** Procédé selon la revendication 1 à 14 dans lequel la phase gazeuse introduite dans le ou les réacteurs contient en mole de 60 % à 99,99 % d'hydrogène le complément à 100% étant un gaz inerte de préférence choisi dans le groupe formé par le méthane, le propane, le butane, l'azote, l'argon, le monoxyde de carbone et le dioxyde de carbone.

**16.** Procédé selon l'une des revendications 12 à 15 dans lequel la proportion de métal noble contenu dans le catalyseur utilisé dans le premier réacteur est inférieure à celle du catalyseur utilisé dans le deuxième réacteur.

**Claims**

**1.** A process for hydrogenating a liquid cut containing hydrocarbons, in particular unsaturated molecules containing at least two double bonds or at least one triple bond, **characterized in that** the unsaturated molecules containing at least two double bonds or at least one triple bond are at least partially hydrogenated into less unsaturated molecules containing at least one double bond, in at least one reactor comprising at least two distinct beds of at least one hydrogenation catalyst, wherein a portion of a gas phase containing hydrogen is introduced as a mixture with said cut upstream of the first catalyst bed and a portion thereof is introduced upstream of the subsequent beds contained in said reactor, wherein in the first reactor hydrogenation is carried out in a liquid and gas downflow mode and wherein the gas/liquid mixture from said first reactor is sent to a separation section from which a gas phase is recovered, an aqueous phase is eliminated and a liquid phase is recovered containing the hydrogenated product from said first reactor at least a portion of which is returned as a mixture with said cut to the inlet to said first reactor upstream of the first catalyst bed contained in said first reactor, and wherein the recycle ratio in the first reactor, equal to the ratio of the volume flow rate of the recycle to the volume flow rate of the feed entering said reactor, is about 10:1 to about 30:1.

**2.** A process according to claim 1, wherein hydrogenation is carried out with a flooded gasliquid flow regime with a volume boil-off rate in the range 5% to 30%.

**3.** A process according to claim 1, wherein hydrogenation is carried out in liquid flow alone, with a boil-off rate in the range 0 to 5%

**4.** A process according to any one of claims 1 to 3, wherein the cut containing the hydrocarbons is a cut essentially comprising hydrocarbons containing butadiene.

**5.** A process according to claim 4, wherein the cut contains butadiene in a proportion by weight of 20% to 95%.

**6.** A process according to any one of claims 1 to 5, wherein the recycle ratio in the first reactor, equal to the ratio of the volume flow rate of the recycle to the volume flow rate of the feed entering said reactor, is about 15:1 to about 25:1.

**7.** A process according to any one of claims 1 to 6, wherein the first reactor contains two successive beds of catalyst and the distribution of the flow rate of the hydrogen-containing gas phase injected as a mixture with the liquid cut into the head of the first reactor upstream of the first catalyst bed is from 50 mole % to 70 mole % with respect to the total molar flow rate of the gas phase injected into the reactor.

**8.** A process according to any one of claims 1 to 7, wherein upstream of said introduction into said reactor, the liquid feed and the gas phase introduced into the head of the first reactor traverse a static mixer.

**9.** A process according to any one of claims 1 to 8, wherein hydrogenation is carried out in two successive reactors, the first operating in liquid and gas downflow mode and the second operating in liquid and gas upflow mode, containing:

- at least two successive beds of catalyst;
- introduction upstream of each of the catalyst beds of a gas phase containing hydrogen corresponding to a portion of the total gas phase introduced into said reactors;
- introduction of a liquid phase corresponding to a portion of the hydrogenated product from said first reactor.

10. A process according to claim 9, wherein the second reactor contains two successive beds of catalyst and the flow rate of the gas phase containing hydrogen injected upstream of the first bed of catalyst is from 50% to 70% with respect to the total molar flow rate of the gas phase injected into said reactor.

11. A process according to any one of claims 1 to 10, wherein the gas phase from the separation section is sent to a chilling section from which a liquid phase is recovered that is recycled to the separation section.

12. A process according to any one of claims 1 to 11, wherein the hydrogenation catalysts used are identical or different in each catalytic bed of each of the reactors and comprise at least one noble group VIII metal on a mineral support, preferably palladium on alumina.

13. A process according to claim 12, wherein the first reactor comprises two identical beds of catalyst containing the same noble group VIII metal on the same mineral support in identical proportions.

14. A process according to claim 12 or claim 13, wherein the catalyst contains a doping agent selected from the group formed by gold, silver and copper in a quantity of about 1 to about 10000 ppm by weight with respect to the support.

15. A process according to any one of claims 1 to 14, wherein the gas phase introduced into the reactor or reactors contains 60 mole % to 99.99 mole % of hydrogen, the complement to 100% being an inert gas, preferably selected from the group formed by methane, propane, butane, nitrogen, argon, carbon monoxide and carbon dioxide.

16. A process according to any one of claims 12 to 15, wherein the proportion of noble metal contained in the catalyst used in the first reactor is less than that of the catalyst used in the second reactor.

**Patentansprüche**

1. Verfahren zur Hydrierung einer flüssigen Fraktion, die Kohlenwasserstoffe und insbesondere ungesättigte Moleküle, die mindestens zwei Doppelbindungen oder mindestens eine Dreifachbindung aufweisen, enthält, **dadurch gekennzeichnet, dass** die ungesättigten Moleküle, die mindestens zwei Doppelbindungen oder mindestens eine Dreifachbindung aufweisen, in mindestens einem Reaktor, der mindestens zwei verschiedene Betten mindestens eines Hydrierkatalysators aufweist, zumindest teilweise zu weniger ungesättigten Molekülen hydriert werden, die mindestens eine Doppelbindung aufweisen, **dadurch**, dass eine Gasphase, die Wasserstoff enthält, teils vermischt mit der Fraktion vor dem ersten Katalysatorbett und teils vor den folgenden Betten, die in dem Reaktor enthalten sind, eingeleitet wird, und **dadurch**, dass in dem ersten Reaktor die Hydrierung im Abwärtsstrom der Flüssigkeit und des Gases durchgeführt wird, und **dadurch**, dass das aus dem ersten Reaktor kommende Gas/Flüssigkeits-Gemisch in einen Trennteil geschickt wird, aus dem eine Gasphase, eine wässrige Phase, die entfernt wird, und eine flüssige Phase, die das aus dem ersten Reaktor kommende hydrierte Produkt enthält und am Einlass des ersten Reaktors zumindest teilweise vermischt mit der Fraktion vor dem ersten Katalysatorbett, das in dem ersten Reaktor enthalten ist, wieder eingeleitet wird, gewonnen werden, und **dadurch**, dass die Rate der Rückführung in den ersten Reaktor, die gleich dem Verhältnis des Volumendurchsatzes der Rückführung zum Volumendurchsatz der Beschickung, die in den Reaktor eingeleitet wird, ist, zwischen 10:1 und 30:1 enthalten ist.

2. Verfahren nach Anspruch 1, wobei sich die Hydrierung im Gas/Flüssigkeits-Strom im Strömungszustand bei einer Volumenverdampfungsrate im Bereich zwischen 5 und 30 % vollzieht.

3. Verfahren nach Anspruch 1, wobei sich die Hydrierung nur im Flüssigkeitsstrom bei einer Verdampfungsrate im Bereich zwischen 0 und 5 % vollzieht.

4. Verfahren nach einem der Ansprüche 1 bis 3, wobei die Kohlenwasserstoffe enthaltende Fraktion eine Fraktion ist, die im Wesentlichen butadienhaltige Kohlenwasserstoffe enthält.

5. Verfahren nach Anspruch 4, wobei die Fraktion Butadien in einem Massenverhältnis von etwa 20 % bis 95 % enthält.

6. Verfahren nach einem der Ansprüche 1 bis 5, wobei die Rate der Rückführung in den ersten Reaktor, die gleich dem Verhältnis des Volumendurchsatzes der Rückführung zum Volumendurchsatz der Beschickung, die in den Reaktor eingeleitet wird, ist, zwischen 15:1 und 25:1 enthalten ist.

7. Verfahren nach einem der Ansprüche 1 bis 6, wobei der erste Reaktor zwei aufeinander folgende Katalysatorbetten enthält und die Aufteilung des Mengendurchsatzes der wasserstoffhaltigen Gasphase, die vermischt mit der flüssigen Fraktion im oberen Teil des ersten Reaktors, vor dem ersten Katalysatorbett eingeblasen wird, etwa 50 bis 70 Mol-%, bezogen auf die Gesamtsumme des Stoffmengendurchsatzes der in den Reaktor eingeblasenen Gasphase beträgt.

8. Verfahren nach einem der Ansprüche 1 bis 7, wobei die flüssige Beschickung und die Gasphase, die beide im oberen Teil des ersten Reaktors eingeleitet werden, vor der Einbringung in den Reaktor einen statischen Mischer durchströmen.

9. Verfahren nach einem der Ansprüche 1 bis 8, wobei sich die Hydrierung in zwei aufeinander folgenden Reaktoren vollzieht, wovon der erste mit einem Abwärtsstrom der Flüssigkeit und des Gases arbeitet und der zweite mit einem Aufwärtsstrom der Flüssigkeit und des Gases arbeitet, umfassend:

   - wenigstens zwei aufeinander folgende Katalysatorbetten,
   - vor jedem der Katalysatorbetten ein Einleiten einer wasserstoffhaltigen Gasphase, die einem Teil der gesamten in die Reaktoren eingeleiteten Gasphase entspricht,
   - ein Einleiten einer flüssigen Phase, die einem Teil des hydrierten Produkts, das aus dem ersten Reaktor kommt, entspricht.

10. Verfahren nach Anspruch 9, wobei der zweite Reaktor zwei aufeinander folgende Katalysatorbetten enthält und der Mengendurchsatz der wasserstoffhaltigen Gasphase, die vor dem ersten Katalysatorbett eingeblasen wird, etwa 50 bis 70 %, bezogen auf den Gesamtstoffmengendurchsatz der in den Reaktor eingeblasenen Gasphase, beträgt.

11. Verfahren nach einem der Ansprüche 1 bis 10, wobei die aus dem Trennteil kommende Gasphase in einen Abkühlungsteil geschickt wird, aus dem eine flüssige Phase gewonnen wird, die wieder in den Trennteil zurückgeführt wird.

12. Verfahren nach einem der Ansprüche 1 bis 11, wobei die verwendeten Hydrierkatalysatoren in jedem Katalysatorbett jedes der Reaktoren identisch oder verschieden sind und auf einem mineralischen Träger mindestens ein Edelmetall der VIII. Gruppe, vorzugsweise Palladium auf Aluminiumoxid, aufweisen.

13. Verfahren nach Anspruch 12, wobei der erste Reaktor zwei identische Katalysatorbetten umfasst, die das gleiche Edelmetall der VIII. Gruppe auf einem gleichen mineralischen Träger in einem identischen Verhältnis aufweisen.

14. Verfahren nach Anspruch 12 oder 13, wobei der Katalysator ein Dotierungsmittel, das aus der Gruppe bestehend aus Gold, Silber und Kupfer gewählt ist, in einer Menge von ungefähr 1 bis ungefähr 10 000 ppm, bezogen auf das Gewicht des Trägers, enthält.

15. Verfahren nach einem der Ansprüche 1 bis 14, wobei die in den Reaktor oder die Reaktoren eingeleitete Gasphase etwa 60 bis 99,99 Mol-% Wasserstoff enthält, wobei die auf 100 % ergänzende Menge ein Inertgas ist, das vorzugsweise aus der Gruppe bestehend aus Methan, Propan, Butan, Stickstoff, Argon, Kohlenmonoxid und Kohlendioxid gewählt ist.

16. Verfahren nach einem der Ansprüche 12 bis 15, wobei der Edelmetallanteil an dem Katalysator, der in dem ersten Reaktor verwendet wird, geringer als jener an dem Katalysator ist, der in dem zweiten Reaktor verwendet wird.

## FIG.1

# FIG.2

# FIG.3

# FIG.4